# EUROPEAN PATENT SPECIFICATION

(11) **EP 1 803 754 B1**
(45) Date of publication and mention of the grant of the patent: **12.05.2010**
(21) Application number: 06256637.7
(22) Date of filing: 29.12.2006
(51) Int. Cl.: C08F 293/00, C09D 153/00, C08L 53/00, A61L 27/54, A61L 27/34, C08F 2/38

(54) **Biologically active block copolymers and coated articles thereof**
Biologisch aktive Blockcopolymere und damit beschichtete Artikel
Copolymères en séquence actifs biologiquement et article revêtu

(30) Priority: 30.12.2005 US 323760
(43) Date of publication of application: 04.07.2007
(73) Proprietor: Cordis Corporation, Miami Lakes, FL 33014 (US)
(72) Inventor: Zhao, Jonathan Z., Belle Mead NJ 08502 (US)
(74) Representative: Belcher, Simon James

(56) References cited:
- WO-A-2005/118018
- US-A- 5 723 344
- US-A1- 2004 091 451
- US-A1- 2005 070 936
- US-A1- 2005 169 957
- VULIC I ET AL: "HEPARIN-CONTAINING BLOCK COPOLYMERS. PART II. IN VITRO AND EX VIVO BLOOD COMPATIBILITY" JOURNAL OF MATERIALS SCIENCE. MATERIALS IN MEDICINE, CHAPMAN AND HALL, LONDON, GB, vol. 4, no. 5, 1993, pages 448-459, XP009051647 ISSN: 0957-4530
- JAGUR-GRODZINSKI J: "Biomedical application of functional polymers" REACTIVE & FUNCTIONAL POLYMERS, ELSEVIER SCIENCE PUBLISHERS BV, NL, vol. 39, no. 2, 15 February 1999 (1999-02-15), pages 99-138, XP004160680 ISSN: 1381-5148

## Description

The present invention relates to a class of block copolymers and a coating composition comprising the said block copolymers. The present invention also relates to an article having the said coating thereon.

Most medical devices are made from metals, ceramics, or polymeric materials. However, these materials are hydrophobic, non-conformal, and non-slippery, and thereby may cause thrombus formation, inflammation, or other injuries to mucous membranes during use or operation. Thus, the issue of biocompatibility is a critical concern for manufacturers of medical devices, particularly medical implants. In order to function properly and safely, medical devices are usually coated with one or more layers of biocompatible materials. The coatings on these medical devices may, in some instances, be used to deliver therapeutic and pharmaceutical agents.

Since medical devices, particularly implantable medical devices, are intended for prolonged use and directly interface with body tissues, body fluids, electrolytes, proteins, enzymes, lipids, and other biological molecules, the coating materials for medical devices must meet stringent biological and physical requirements. These requirements, as a minimum, include the following: (1) the coatings must be hydrophilic and lubricous when in contact with body tissue, and thereby increase patient comfort during operation and enhance the maneuverability of the medical device; (2) the coatings must be flexible and elastic, so they conform to the biological structure without inducing detrimental stress; (3) the coatings must be hemocompatible, and thereby reduce or avoid formation of thrombus or emboli; (4) the coatings must be chemically inert to body tissue and body fluids; and (5) the coatings must be mechanically durable and not crack when formed on medical devices. If the coatings are impregnated with pharmaceutical or therapeutic agents, it is typically required that the coatings and the formation thereof are compatible with the pharmaceutical or therapeutic agents. If the coatings are used as coatings and the underlying basecoats are impregnated with pharmaceutical or therapeutic agents, it is further required that the coating and the formation thereof must be compatible with the basecoat and the pharmaceutical or therapeutic agents impregnated therein; and the coating must allow the pharmaceutical or therapeutic agents to permeate therethrough. It is also desirable that the coating functions as a physical barrier, a chemical barrier, or a combination thereof to control the elution of the pharmaceutical or therapeutic agents in the underlying basecoat.

In order to combine the desired properties of different polymeric materials, the conventional coating composition for commercial drug eluting stents used a polymer blend, i.e., physical mixture, of poly ethylene-vinyl acetate (EVAc) and poly butyl methacrylate (BMA). However, one disadvantage of this conventional coating is the phase separation of the polymer blend, which can be detrimental to the performance of the coating and the stability of drugs impregnated therein.

Another coating composition of the prior art comprises a supporting polymer and a hydrophilic polymer, wherein the supporting polymer contains functional moieties capable of undergoing crosslinking reactions and the hydrophilic polymer is associated with the supporting polymer (see, for example, US-6238799). However, the preparation of this prior art coating composition employs chemical crosslinking reactions and a high temperature curing process, which are not compatible with a drug-containing coating.

The prior art also uses a coating composition formed by the gas phase or plasma polymerization of a gas comprising monomers of polyethylene glycol vinyl ether compounds (see, for example, US-A-2003/0113477). However, the polymer prepared through the plasma process has poorly defined molecular weight and a large polydispersity. The plasma laid polymers of low molecular weight have limited mechanical durability. Further, plasma treatment can penetrate through the underlying basecoat and damage the drug content therein. Another problem with this prior art approach is that the free radicals or other high energy species generated in the plasma process may persist in the coating and cause drug content loss in the basecoat over time.

To decrease thrombosis caused by the use of medical devices, the prior art also modifies the coatings of medical devices via conjugating, i.e., covalently bonding, an antithrombotic agent (e.g., heparin) to the coatings (see, for example, US-4,973,493 and www.surmodics.com). Although this approach may produce a coating with excellent antithrombotic property, the prior art conjugation methods employ complex preparation processes and produce various by-products that may cause degradation of the antithrombotic agent in the coating.

Vulic et al have disclosed in their paper "Heparin-containing block copolymers" (J Mater Sci Mater Med 4 (1993) 448-459) a block copolymer which comprises a polystyrene block, a polyethylene glycol block and heparin.

Jagur-Grodzinski has disclosed in his paper "Biomedical application of functional polymers" (React Funct Polym 39 (1999) 99-138) a block copolymer which comprises a polyetherurethane urea block, a polyacrylic acid block, and a heparin block.

US-A-2005/169957 discloses a block copolymer comprising a polybutylmethacrylate block, and polyethylene glycol block.

US-A-2004/091451 discloses a polymer material having a hydrophobic block such as an acrylate and a hydrophilic block such as N-vinyl pyrrolidone, and which has ligand groups fixed to it. The ligand groups can be selected for their ability to target a biologically active molecule, for example for use in diagnosis.

US-A-2005/070936 discloses a polymeric material for use as a coating on a medical device. The material is based on an ethylene-vinyl alcohol copolymer, having additional adducts introduced into its backbone chain, for example through reaction with a heparin.

Thus, there remains a need for a polymeric material and a coating composition that can satisfy the stringent requirements, as described above, for applying on at least one surface of a medical device and can be prepared through a process that is compatible with the pharmaceutical or therapeutic agents physically or chemically impregnated in the coatings.

The present invention provides a block copolymer as defmed in claim 1.

The present invention also provides a coating composition for applying on at least a portion of one surface of an article, said coating composition comprising a block copolymer as defined in claim 1.

In another aspect, the present invention provides an article having a coating thereon, said coating comprising a block copolymer as defined in claim 1

The present invention provides a block copolymer comprising a hydrophobic block, a hydrophilic block, and a biologically active block. The biologically active block is directly adjacent to the hydrophilic block. By "block copolymer", it is meant a heteropolymer comprising blocks of different polymerized monomers. Preferably, the inventive block copolymer is linear. That is, it is preferred that the inventive block copolymer has a shape of a straight chain.

By "hydrophobic", it is meant lacking affinity for water and tending to dissolve in or mix with organic solvents or lipids. During polymerization, the vinyl moieties of the monomer units of one or more alkyl methacrylate or alkyl acrylate form a linear backbone, while the moieties other than the vinyl moieties of the monomer units of one or more alkyl methacrylate or alkyl acrylate constitute pendant groups covalently attached to the linear backbone. By "alkyl methacrylate", it is meant a methacrylate derivative wherein the oxygen atom attached to the carbon atom of the carbonyl group is substituted with an alkyl group. By "alkyl acrylate", it is meant an acrylate derivative wherein the oxygen atom attached to the carbon atom of the carbonyl group is substituted with an alkyl group.

By "hydrophilic" it is meant having a strong affinity for water and tending to dissolve in, mix with, or swell in water or aqueous medium. During polymerization, the vinyl moieties of the monomer units selected from the group described above form a linear backbone, while the moieties other than the vinyl moieties of the monomer units selected from the group described above constitute pendant groups covalently attached to the linear backbone.

The biologically active block of the inventive block copolymer comprises a linear backbone derived from vinyl moieties and pendantbiologically active molecules. By "linear backbone derived from vinyl moieties", it is meant the backbone of the biologically active block is a straight chain and is formed by polymerization of vinyl groups. The pendantbiologically active molecules are covalently attached to the linear backbone derived from vinyl moieties. The "pendant biologically active molecule" as used herein denotes a compound or substance having an effect on or eliciting a response from living tissue. The pendant biologically active molecules suitable for the present invention include, for example, any drugs, agents, compounds and/or combination thereof that have therapeutic effects for treating or preventing a disease or a biological organism's reaction to the introduction of the medical device to the organism. Preferred pendant biologically active molecules include, but are not limited to: anti-thrombogenic agents, immuno-suppressants, anti-neoplastic agents, anti-inflammatory agents, angiogenesis inhibitors, protein kinase inhibitors, and other agents which may cure, reduce, or prevent restenosis in a mammal. Preferred pendant biologically active molecules also include proteins, peptides, DNA, RNA, siRNA, ribozymes, polysaccharides, oligosaccharides, and lipids. Examples of the pendant biologically active molecules of the present invention include, but are not limited to: heparin, albumin, streptokinase, tissue plasminogin activator (TPA), urokinase, rapamycin, paclitaxel, pimecrolimus, proteins, peptides, DNA, RNA, siRNA, ribozymes, polysaccharides, oligosaccharides, lipids, and their analogs and derivatives. Preferably, the heparin used in the present invention is a low molecular weight heparin. The biologically active block imparts biological activity to the inventive block copolymer. Since a wide range of pendant biologically active molecules can be used for the biologically active block, the biological activity of the inventive block copolymer may be adjusted accordingly.

The inventive block copolymer may be prepared through living polymerization methods. More preferably, the inventive block copolymer is prepared through reversible addition fragmentation transfer (RAFT) polymerization. Many conventional polymerization methods require chemical crosslinking reactions, high temperature curing processes, and/or plasma treatments, which not only have very limited control over the polymer molecular weight distribution, but also cause damages to the therapeutic agent impregnated in the coating and the drug-content in the underlying basecoat. Unlike those conventional polymerization methods, RAFT polymerization allows precise control of the molecular weight and molar ratio of each segment of a copolymer at ambient temperature, thereby providing a copolymer with predetermined molecular weight and narrow polydispersity, i.e., narrow molecular weight distribution. Thus, the structure and the molecular weight of the inventive block copolymer may be precisely tuned through employment of RAFT polymerization.

Accordingly, the properties of the inventive block copolymer may be tuned via adjusting the structure and/or the molar ratios of the hydrophobic block, the hydrophilic block, and the biologically active block. In other words, the structure and/or the molar ratios of the hydrophobic block, the hydrophilic block, and the biologically active block may be adjusted according to the desired properties of the inventive block copolymer. For example, the hydrophilicity or hydrophobicity of the inventive block copolymer may be adjusted through the use of hydrophilic block and/or hydrophobic block having different repeating monomer units, and/or through controlling the molar ratio between the hydrophobic block and the hydrophilic block. Furthermore, the hydrophobic block, the hydrophilic block, and the biologically active block need to be in a molar ratio that ensures desired mechanical strength of the inventive block copolymer while providing a hydrophilic environment for retaining the optimal activity of the biologically active block. Preferably, the copolymer has the hydrophobic block, the hydrophilic block, and the biologically active block in a mole ratio of 1:1:1.

In one embodiment of the present invention, the inventive block copolymer having structure (II) is synthesized through a route illustrated in Scheme 1. 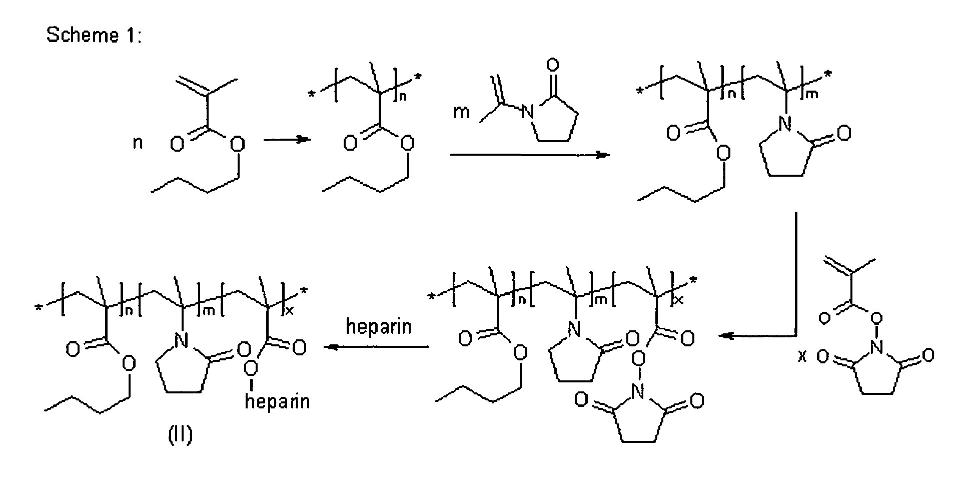 wherein x, n, and m are the same or different, and are independently an integer of 10 to 2500. RAFT polymerization has been reported in recent literatures, and one skilled in the art would be able to readily ascertain details of RAFT reaction conditions (see, for example, Shi, Peng-Jie; et al. European Polymer Journal, 2004, 40, 1283-1290).

Various functional blocks can be added to the inventive block copolymer via employing RAFT polymerization. Thus, the properties of the inventive block copolymer may be tuned accordingly. In one embodiment of the present invention, the inventive block copolymer may further comprise a photoactive block. It is preferred that the photoactive block is directly adjacent to the hydrophobic block. The photoactive block comprises a linear backbone derived from vinyl moieties and pedant photoreactive molecules. By "linear backbone derived from vinyl moieties", it is meant the backbone of the biologically active block is a straight chain and is formed by polymerization of vinyl groups. The pendant photoreactive molecules are covalently attached to the linear backbone derived from vinyl moieties. By "pendant photoreactive molecules", it is meant molecules that absorb ultraviolet light of certain wavelength band and consequently initiate a crosslinking polymerization process. The pendant photoreactive molecules may be any photoreactive molecules compatible with the hydrophobic block, the hydrophilic block, and the biologically active block of the inventive block copolymer. Examples of pendant photoreactive molecules suitable for the present invention include, but are not limited to: benzophenone, azide, thioxanthone, and derivatives thereof.

In one embodiment of the present invention, the inventive block copolymer comprises the following structure (structure III): 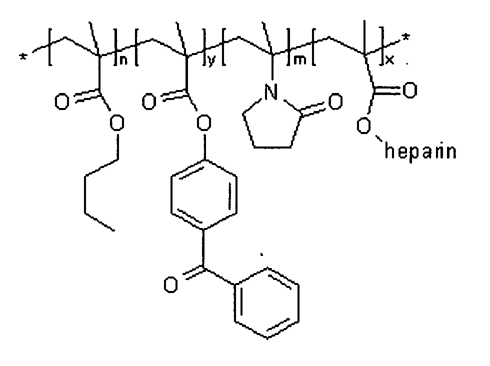 wherein x, n, and m are the same or different, and are independently an integer of 10 to 2500, and y is an integer of 1 to 10.

In one embodiment of the present invention, the inventive block copolymer of structure (III) is synthesized through a route illustrated Scheme 2: 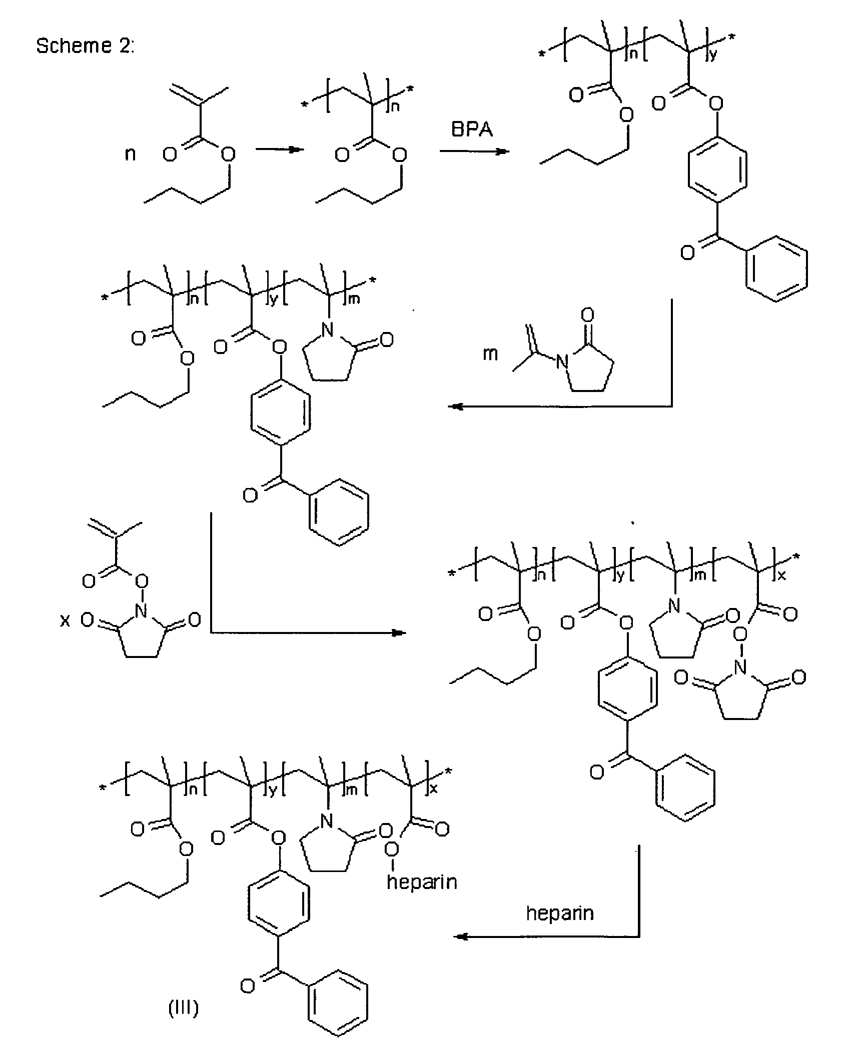 wherein x, n, and m are the same or different, and are independently an integer of 10 to 2500, and y is an integer of 1 to 10; and BPA is benzophenone methacrylate, which has the following structure (structure VI):

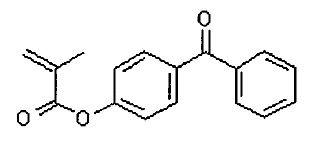

It is preferable that the inventive block copolymer has a tunable polymer molecular weight ranging from about 5,000 to about 500,000 Daltons to enable the formation of a coating with desirable mechanical durability and adequate adhesiveness. Since the mechanical durability of a coating improves upon increasing polymer molecular weight, it is especially preferable that the inventive block copolymer has a high polymer molecular weight of 10,000 to 500,000 Daltons for use in coatings for certain medical devices (e.g., stents) which require expansion and deployment in vivo.

The present invention also provides a coating composition for applying on at least a portion of one surface of an article. The coating composition comprises a block copolymer having a hydrophobic block, a hydrophilic block, and a biologically active block, wherein the biologically active block is directly adjacent to the hydrophilic block. Preferably, the block copolymer is linear. The pendant biologically active molecules are covalently attached to the linear backbone derived from vinyl moieties. Preferred pendant biologically active molecules include, but are not limited to: anti-thrombogenic agents, immuno-suppressants, anti-neoplastic agents, anti-inflammatory agents, angiogenesis inhibitors, protein kinase inhibitors, and other agents which may cure, reduce, or prevent restenosis in a mammal. Preferred pendant biologically active molecules also include proteins, peptides, DNA, RNA, siRNA, ribozymes, polysaccharides, oligosaccharides, and lipids. Examples of the pendant biologically active molecules of the present invention include, but are not limited to: heparin, albumin, streptokinase, tissue plasminogin activator (TPA), urokinase, rapamycin, paclitaxel, pimecrolimus, proteins, peptides, DNA, RNA, siRNA, ribozymes, polysaccharides, oligosaccharides, lipids, and their analogs and derivatives. Preferably, the heparin used in the present invention is a low molecular weight heparin.

The block copolymer may further comprise a photoactive block. It is preferred that the photoactive block is directly adjacent to the hydrophobic block. The photoactive block comprises a linear backbone derived from vinyl moieties and pendant photoreactive molecules. The pendant photoreactive molecules are covalently attached to the linear backbone derived from vinyl moieties. The pendant photoreactive molecules may be any photoreactive molecules compatible with the hydrophobic block, the hydrophilic block, and the biologically active block of the inventive block copolymer. Examples of pendant photoreactive molecules suitable for the present invention include, but are not limited to: benzophenone, azide, thioxanthone, and derivatives thereof. The pendant photoactive block allows photo crosslinking of the inventive block copolymer, thereby enhancing the durability of the inventive coating composition.

The inventive coating composition may additionally include co-solvents and/or other additives to facilitate high quality film formation, such as plasticizers, antifoaming agents, anticrater agents, and coalescing solvents. Other suitable additives to the inventive coating composition include, but are not limited to: bioactive agents, antimicrobial agents, antithrombogenic agents, antibiotics, pigments, radiopacifiers and ion conductors. Details concerning the selection and amounts of such ingredients are known to those skilled in the art.

The inventive coating composition may be applied on at least a portion of one surface of an article. In some embodiments, the inventive coating is applied to all exposed surfaces of an article. The thickness of the inventive coating composition may vary depending on the process used in forming the coating as well as the intended use of the article. Typically, and for a medical device, the inventive coating is applied to a thickness from about 1 nanometer to about 10 micrometer, with a thickness from about 10 nanometer to about 10 micrometer being more typical. The inventive block copolymer is soluble in common organic solvents, such as tetrahydrofuran (THF), acetone, chloroform, dichloromethane, acetonitrile, dimethylformide (DMF), and mixtures thereof. Since organic solvents are widely used to handle polymeric material, the inventive coating composition may be applied on at least one surface of an article through various coating processes (e.g., spray coating process).

When applied on at least one surface of an article, the linear backbone and the hydrophobic block provide the inventive block copolymer with improved mechanical durability and enhanced adhesion to the underlying surface, while the hydrophilic block and the biologically active block impart lubricity and hemocompatibility. Furthermore, the hydrophobic block and the hydrophilic block are adjustable to various lengths to obtain the desirable elasticity of the inventive block copolymer. Moreover, the hydrophilic block can hydrate and swell under physiological conditions and provide a desirable environment for the biologically active block to retain the biological activity.

The inventive coating composition may also be applied to control the elution of a therapeutic dosage of a pharmaceutical agent from a medical device base coating, for example, a stent base coating. The basecoat generally comprises a matrix of one or more drugs, agents, and/or compounds and a biocompatible material such as a polymer. The control over elution results from either a physical barrier, or a chemical barrier, or a combination thereof. The elution is controlled by varying the thickness of the coating, thereby changing the diffusion path length for the drugs, agents, and/or compounds to diffuse out of the basecoat matrix. Essentially, the drugs, agents and/or compounds in the basecoat matrix diffuse through the interstitial spaces in the coating. Accordingly, the thicker the coating, the longer the diffusion path, and conversely, the thinner the coating, the shorter the diffusion path. The effectiveness of the inventive coating composition as a regulator for drug elution from the basecoat may be maximized via tuning the relative molar ratio of the various blocks in the block copolymer for the optimal hydrophobicity of the block copolymer. It is important to note that both the basecoat and the coating thickness may be limited by the desired overall profile of the article on which they are applied.

The present invention also provides an article having a coating thereon. The at least a portion of one surface of the article may be a surface of a polymeric coat, a plastic substance, ceramic, steel, or other alloy metals. Various functional blocks, such as, for example, a photoactive block, can be added to the inventive block copolymer to impart desirable properties to the inventive block copolymer and the inventive coating.

The article that may be coated with the inventive coating composition may be in any shape, and is preferably a medical device or a component of a medical device. More preferably, the medical device or the component of a medical device is implantable. The term "medical device" as used herein denotes a physical item used in medical treatment, which includes both external medical devices and implantable medical devices. The medical devices that may be coated with the inventive coating composition include, but are not limited to: catheters, guidewires, drug eluting stents, cochlear implants, retinal implants, gastric bands, neurostimulation devices, muscular stimulation devices, implantable drug delivery devices, intraocular devices, and various other medical devices.

The present coating composition may be applied to the surface of an article using conventional coating techniques, such as, for example, spray coating, ultrasonic coating, dip coating, and the like. In a dip coating process, the article is immersed in a bath containing the coating composition and then removed. A dwelling time ranging from 1 minute to 2 hours may be used depending of the material of construction, complexity of the device, and the desired coating thickness. Next, the article coated with the coating composition may be allowed to dry to provide a dry coating. Drying may be accomplished merely by standing at ambient conditions or may be accelerated by heating at mild temperatures, such as 30°C to 65°C.

## Claims

1. A block copolymer comprising a hydrophobic block, a hydrophilic block, and a biologically active block, wherein the biologically active block is directly adjacent to the hydrophilic block and comprises a linear backbone derived from vinyl moieties and pendant biologically active molecules, the pendant biologically active molecules are covalently attached to the linear backbone derived from vinyl moieties, the block copolymer having the following structure: 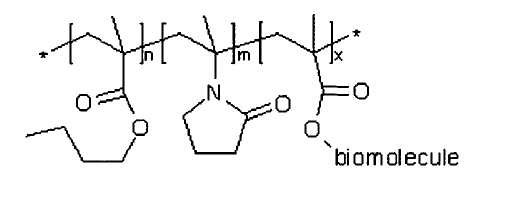 wherein x, n, and m are the same or different, and are independently an integer of 10 to 2500; and biomolecule is selected from the group consisting of anti-thrombogenic agents, immuno-suppressants, anti-neoplastic agents, anti-inflammatory agents, angiogenesis inhibitors, protein kinase inhibitors, proteins, peptides, DNA, RNA, siRNA, ribozymes, polysaccharides, oligosaccharides, and lipids.

2. A block copolymer as claimed in claim 1, which has the following structure: 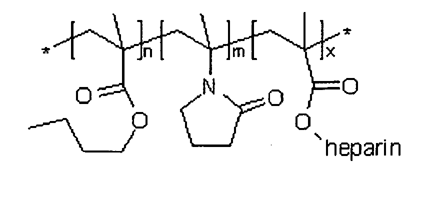 wherein x, n, and m are the same or different, and are independently an integer of 10 to 2500.

3. A block copolymer as claimed in claim 1, which includes a photoactive block, said photoactive block comprising a linear backbone derived from vinyl moieties and pendant photoreactive molecules, the pendant photoreactive molecules are covalently attached to the linear backbone derived from vinyl moieties.

4. The block copolymer of claim 3, wherein the pendant photoreactive molecules are benzophenone, azide, thioxanthone, or derivatives thereof.

5. The block copolymer of claim 4, which comprises the following structure: 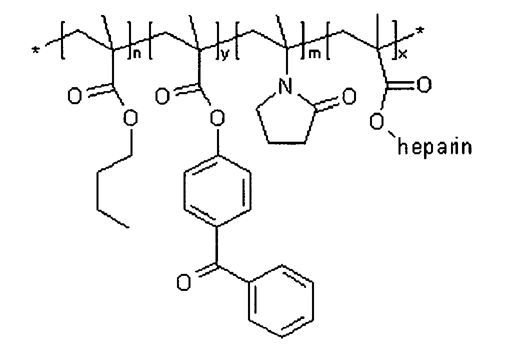 wherein x, n, and m, are the same or different, and are independently an integer of 10 to 2500; and y is an integer of 1 to 10.

6. A medical device having a coating applied to it formed from a block copolymer as claimed in claim 1.

## Patentansprüche

1. Blockcopolymer, welches einen hydrophoben Block, einen hydrophilen Block und einen biologisch aktiven Block umfasst, wobei der biologisch aktive Block dem hydrophilen Block direkt benachbart ist und ein lineares Rückgrat, welches von Vinylresten abgeleitet ist, und seitenständige biologisch aktive Moleküle umfasst, wobei die seitenständigen biologisch aktiven Moleküle kovalent an das lineare Rückgrat gebunden sind, welches von Vinylresten abgeleitet ist, wobei das Blockcopolymer die folgenden Struktur aufweist: 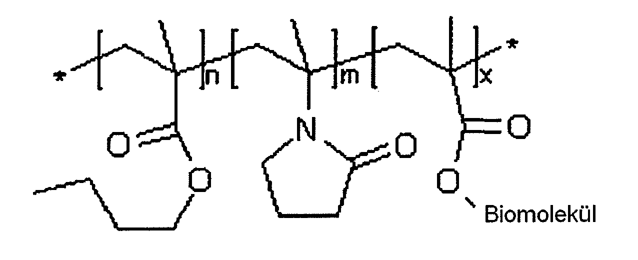 wobei x, n und m gleich oder unterschiedlich sind und unabhängig eine Ganzzahl von 10 bis 2500 sind, und wobei das Biomolekül ausgewählt ist aus der Gruppe, welche aus antithrombogenen Wirkstoffen, Immunsuppressiva, antineoplastischen Wirkstoffen, enzündungshemmenden Wirkstoffen, Angiogenesehemmern, Proteinkinasehemmem, Proteinen, Peptiden, DNA, RNA, siRNA, Ribozymen, Polysacchariden, Oligosacchariden und Lipiden besteht.

2. Blockcopolymer gemäß Anspruch 1, welches die folgende Struktur aufweist: 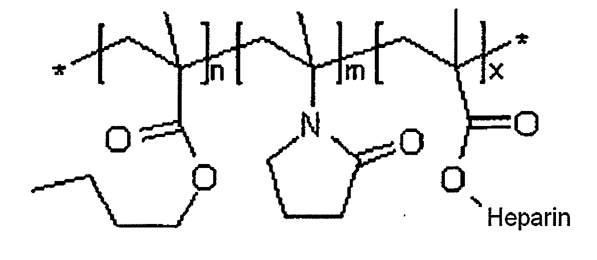 wobei x, n und m gleich oder unterschiedlich sind und unabhängig eine Ganzzahl von 10 bis 2500 sind.

3. Blockcopolymer gemäß Anspruch 1, welches einen fotoaktiven Block aufweist, wobei der fotoaktive Block ein lineares Rückgrat, welches von Vinylresten abgeleitet ist, und seitenständige fotoreaktive Moleküle umfasst, wobei die seitenständigen fotoreaktiven Moleküle kovalent an das lineare Rückgrat gebunden sind, welches von Vinylresten abgeleitet ist.

4. Blockcopolymer nach Anspruch 3, wobei die seitenständigen fotoreaktiven Moleküle Benzophenon, Azid, Thioxanthon oder Derivate davon sind.

5. Blockcopolymer nach Anspruch 4, welches die folgende Struktur umfasst: 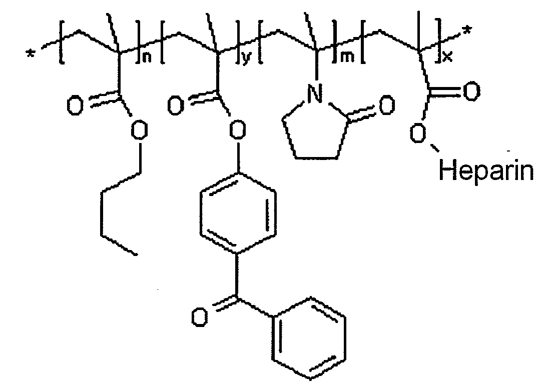 wobei x, n und m gleich oder unterschiedlich sind und unabhängig eine Ganzzahl von 10 bis 2500 sind; und wobei y eine Ganzzahl von 1 bis 10 ist.

6. Medizinische Vorrichtung, welche eine darauf applizierte Beschichtung aufweist, welche aus einem Blockcopolymer gemäß Anspruch 1 gebildet ist.

## Revendications

1. Copolymère à blocs comprenant un bloc hydrophobe, un bloc hydrophile et un bloc biologiquement actif, dans lequel le bloc biologiquement actif est directement adjacent au bloc hydrophile et comprend un squelette linéaire dérivé de groupements vinyliques et des molécules biologiquement actives pendantes,les molécules biologiquement actives pendantes sont fixées de manière covalente au squelette linéaire dérivé des groupements vinyliques, le copolymère à blocs ayant la structure suivante : 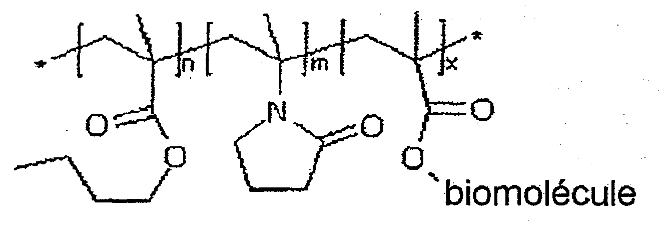 dans laquelle x, n, et m sont identiques ou différents et sont, indépendamment, un entier de 10 à 2 500 ; et la biomolécule est choisie dans le groupe constitué par les agents anti-thrombogènes, les immunosuppresseurs, les agents anti-néoplasiques, les agents anti-inflammatoires, les inhibiteurs de l'angiogenèse, les inhibiteurs de protéine kinase, les protéines, les peptides, l'ADN, l'ARN, les siRNA, les ribozymes, les polysaccharides, les oligosaccharides, et les lipides.

2. Copolymère à blocs selon la revendication 1, qui a la structure suivante ; 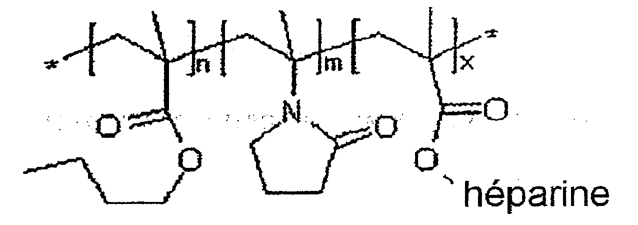 dans laquelle x, n, et m sont identiques ou différents et sont, indépendamment, un entier de 10 à 2 500.

3. Copolymère à blocs selon la revendication 1, qui comprend un bloc photoactif, ledit bloc photoactif comprenant un squelette linéaire dérivé de groupements vinyliques et de molécules photoréactives pendantes, les molécules photoréactives pendantes sont fixées de manière covalente au squelette linéaire dérivé des groupements vinyliques.

4. Copolymère à blocs selon la revendication 3, dans lequel les molécules photoréactives pendantes sont la benzophénone, l'azide, la thioxanthone ou leurs dérivés.

5. Copolymère à blocs selon la revendication 4, qui comprend la structure suivante : 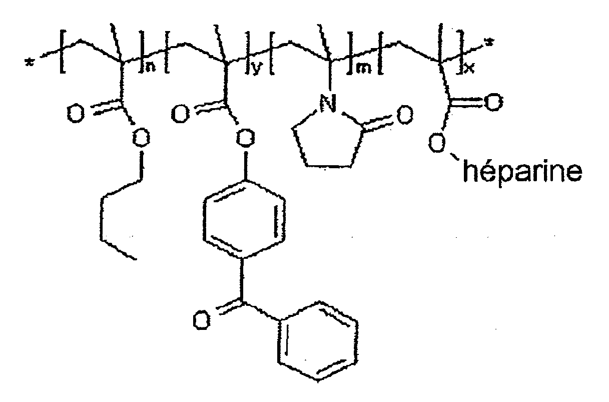 dans laquelle x, n, et m sont identiques ou différents et sont, indépendamment, un entier de 10 à 2 500 et y est un entier de 1 à 10.

6. Dispositif médical comprenant un revêtement qui y a été appliqué, formé à partir d'un copolymère à blocs selon la revendication 1.
